(19) Europäisches Patentamt / European Patent Office / Office européen des brevets

(11) **EP 3 740 585 B1**

(12) **EUROPEAN PATENT SPECIFICATION**

(45) Date of publication and mention
of the grant of the patent:
**27.09.2023  Bulletin 2023/39**

(21) Application number: **19741441.0**

(22) Date of filing: **16.01.2019**

(51) International Patent Classification (IPC):
$C12Q\ 1/04^{(2006.01)}$     $C12Q\ 1/06^{(2006.01)}$
$G01N\ 33/569^{(2006.01)}$     $G01N\ 33/58^{(2006.01)}$
$C12M\ 1/34^{(2006.01)}$     $G01N\ 21/64^{(2006.01)}$
$C12Q\ 1/10^{(2006.01)}$

(52) Cooperative Patent Classification (CPC):
**C12Q 1/06; C12Q 1/045; C12Q 1/10; G01N 33/582;**
G01N 21/94; G01N 2021/6439

(86) International application number:
**PCT/IL2019/050064**

(87) International publication number:
**WO 2019/142187 (25.07.2019 Gazette 2019/30)**

(54) **IMPROVING DETECTION OF MICROORGANISMS**

VERBESSERUNG DER DETEKTION VON MIKROORGANISMEN

AMÉLIORATION DE LA DÉTECTION DE MICRO-ORGANISMES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **17.01.2018   US 201862618094 P**

(43) Date of publication of application:
**25.11.2020  Bulletin 2020/48**

(73) Proprietor: **BACTOBYTE LTD.**
**9655222 Jerusalem (IL)**

(72) Inventor: **GLUKHMAN, Vladimir**
**9655222 Jerusalem (IL)**

(74) Representative: **Zwicker, Jörk**
**ZSP Patentanwälte PartG mbB**
**Hansastraße 32**
**80686 München (DE)**

(56) References cited:
**EP-B2- 0 254 771          WO-A1-89/04372
WO-A1-2005/012555      WO-A1-2016/210436
US-A1- 2005 202 518    US-A1- 2007 281 291
US-A1- 2010 062 466**

**Description**

**FIELD OF INVENTION**

The current invention generally pertains to the use of X-glucuronide, B-galactose and D-Glucose for increasing the intracellular concentration of at least on fluorescent marker molecule selected from the group consisting of Fluorescein, Fluorescein di(β-D-glucuronide), NileRed, and CY5 in Coliform bacteria in a method for rapid detection and quantification of live and recovered Coliform bacteria in a sample.

**BACKGROUND OF INVENTION**

Bacterial contamination and infection is a significant problem to public health, food, industry, environmental biosafety, and many other areas.

**Microorganisms in blood and in sterile fluids:** Human biological fluids are frequently obtained from patients showing symptoms of infection in the corresponding body area to isolate and identify the etiological agent. Samples of this kind are also taken to detect the presence of microorganisms in at-risk patients showing particular pathological conditions (patients subjected to abdominal surgery, ascites in cirrhotic patients, hematological disorders, etc.).

The recovery of microorganisms from blood is crucial for proper diagnosis and treatment of infection. To obtain accurate results, it is necessary to maximize the number of organisms collected from a given sample. This is challenging due to the fact that the concentrations of pathological organisms in the blood vary enormously. One example of this wide range of concentrations is the case of bacteremia, a condition where viable bacteria are present in the circulating blood.

The prompt diagnosis and treatment of bacteremia is of significant interest to health care professionals. When left undiagnosed, bacteremia can lead to systemic inflammatory response syndrome (SIRS) and patients are often at high risk for developing sepsis, the leading cause of death in critically ill patients in Europe and the U.S.A.

Sepsis is a life-threatening condition caused by the uncontrolled, systemic, inflammatory response to bacterial, viral or fungal infection. Sepsis represents a substantial health burden. The incidence of sepsis and the number of sepsis-related deaths are increasing, due to a variety of reasons attributed to the aging of the population, the increasing longevity of patients with chronic diseases, the increasingly aggressive cancer therapies and the increasing use of invasive devices, like cardiac pacemakers, valves and defibrillators, and procedures for a variety of medical conditions are as well as the widespread use of broad-spectrum antibiotics which has increased the rates of both antibiotic resistance and nosocomial fungal infections.

Consequently, it is necessary to develop quick and accurate diagnostics for detecting bacteria in blood, urine, and other normally sterile fluids. Furthermore, rapid and accurate identification of sepsis and its causative organisms are a prerequisite for successful therapy. Delayed recognition of sepsis and inappropriate initial antibiotic therapy are associated with an increase in mortality and morbidity. The current gold standard for the diagnosis of sepsis is culture of blood and other body fluids or tissues. However, even in severe sepsis, blood cultures yield the causative microorganism in only 20-40% of patients. Moreover, at least 24 hours are needed to get preliminary information about the potential organism.

**Detection of microorganisms in pharmaceutical preparations:** Traditional methods to assure quality and detect any microbial contaminants in intravenous products takes at least 2 weeks to complete. Hospital-prepared intravenous products are often high risk and have short shelf lives. This sometimes means that the traditional quality microbiological results are only available after the product has been administered to the patient. As a consequence, there have been some fatal incidents in which contaminated intravenous products have been used.

**Detection of Foodborne Pathogens:** The detection and enumeration of pathogens in food and on surfaces that come into contact with food are an important component of any integrated program to ensure the safety of foods throughout the food supply chain. Both government authorities and food companies use microbiological analysis to monitor the state of contamination at all times and analyze its trends so as to detect emerging risks. Traditional culture methods for detecting microorganisms in food can be laborious and may require several days before results are known. Products that are minimally processed have an inherently short shelf life, which prevents the use of many of these conventional methods.

**Waterborne pathogens:** Waterborne disease is a global burden, while morbidity and mortality caused by contaminated water are enormous and need to be controlled by improving the security of drinking water.

Waterborne infections are caused by ingestion, airborne or contact with contaminated water by a variety of more than 1400 species of infectious agents which includes bacteria, viruses, protozoa fungi and helminths, which may lead to diarrhea, gastrointestinal diseases and systematic illnesses, and even death. It is estimated that 3.2% of deaths globally are attributable to unsafe water caused by poor sanitation and hygiene.

Detection methods play a major role in monitoring water quality, surveillance, and quantitative microbial risk assessment; thus, have a major influence on implementing the best practices to alleviate and prevent threats that allow achieving the goal of water safety.

Bacterial contamination and infection is a significant problem to public health, food, industry, environmental biosafety, and many other areas. However, current methods for detecting bacteria in medical, veterinary, agricultural, food processing, industrial and other contexts are slow, require specialized personnel or equipment to execute, and are often expensive. There is a large unmet need for technologies that can provide quick, sensitive, and specific detection of pathogens to enable proactive, convenient, and rapid safety programs that reduce costs and threats to human health.

US 2005/202518 discloses a method for detecting and counting micro-organisms in a sample by fluorescently labeling the concentrated micro-organism, and detecting and analyzing the fluorescence.

US 2010/062466 discloses a medium for the detection and/or identification of bacteria comprising a substrate for a metabolic activity specific for a group of Gram-negative bacteria.

US 2007/281291 discloses a method of detecting E. coli bacteria in a sample material comprising exposing the induction medium to long wavelength light, and observing fluorescence indicating the presence of E. coli.

EP 0 254 771 discloses a specific medium for combination with a specimen sample to determine the presence of absence of a target microbe in the specimen sample.

WO 89/04372 discloses a process for assaying, in a brief period of time, a dilute concentration of living pathogenic microorganisms in a sample of product for human consumption.

WO 2016/210436 discloses a Salmonella-selective medium comprising fructose-asparagine as a nutrient source, and a method of selecting an isolated bacterial strain that can utilize fructose-asparagine as a nutrient source.

WO 2005/012555 discloses an induction solution which provides for rapid detection of coliforms, capable to induce, in absence of cell growth, the expression of inducible enzymes β-glucuronidase and β-galactosidase.

## BRIEF DESCRIPTION OF THE FIGURES

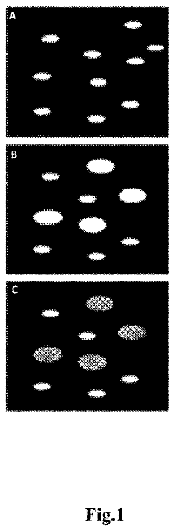

**Fig. 1:** Intracellular concentration of marker molecule of cellular metabolism as a result of the influence of metabolism activators (Scheme)

Illustrations showing the effect of bacterial recovery or metabolism stimulators/activators on dyes concentrations of marker molecules in various cells: Marker molecules dyes increase their concentration (Large white ovals) in recoverable cells with increased metabolism (B). The concentration is low (Small white ovals) in non-recoverable cells (A; B and C): In non- growth and anabiotic microorganisms which have low level of metabolism, the dyes remain confined to the extracellular space. Then recovered cells that additionally labeled with markers (pattern ovals) for species identification by double fluorescent staining and imaging (C).

**Fig. 2:** Morphology of tested *Coliform* bacteria.

**Fig. 3:** Intracellular concentration of markers of cellular metabolism was observed in recovered/metabolism-activated Total *Coliforms* (A) and *Ps.aeruginosa* (B) cultures. Significantly low markers concentration was observed in metabolically shocked *Coliforms* (C) and *A.hydrophilia* treated by unspecific activators.

**Fig. 4:** Defining of initial sample contamination.

Bacterial counts performed by heterotrophic plate count method (normalized to 1ml volume, Axis Y) and by the new method (raw count, Axis X) for *E. coli* (A); *Ps. aeruginosa* (B) and total heterotrophic bacteria (C) are plotted to get trendlines and equations. This process revealed formulas for calculating of initial contamination counts.

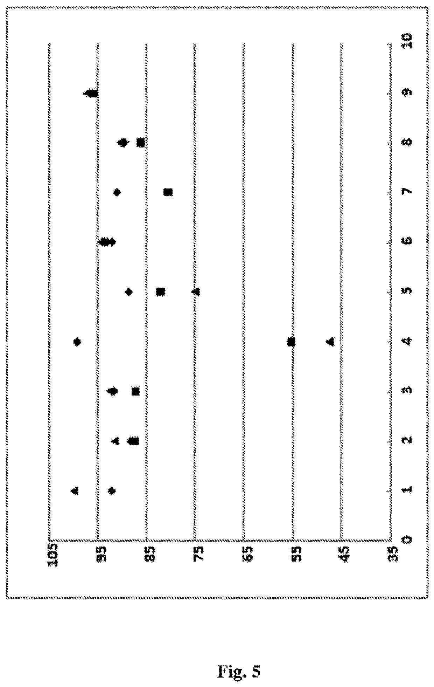

**Fig. 5** Correlation of microbial counts between the tested and conventional methods.

In most cases the correlation between the new and conventional method is defined as about 90%. The correlations were tested by counts of *E.coli* (rhombus), *Ps.aeruginosa* (square) and total heterotrophic bacterial count (triangle).

X and Y axis represent number of experimental series [hr.] and ration of numerical correlation ratio [%] respectively.

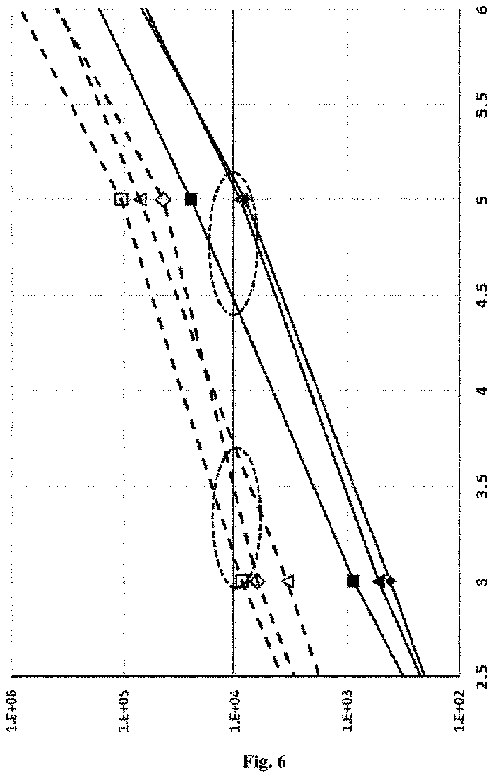

**Fig. 6;** Media containing growth activators enables commencing of bacterial detection 20-80% sooner than standard growth media. Solid horizontal line specifies levels of specific bacteria contamination that are sufficient to reliable microbial detection and enumeration by the new technology.

The lines on the graph are the growth curves of *E.coli* in the newly invented (square) and standard peptone water (triangle) or MacConkey (rhombus) growing broths. The increase in growth rate (marked by oval shapes) is verified for initial concentrations of 10 CFU/ml. (solid lines) and 100 CFU/ml (dotted line).

X and Y axis represent incubation time [hr.] and bacterial concentration [CFU/ml] respectively.

## SUMMARY

The present invention pertains to the use of X-glucuronide, B-galactose and D-Glucose for increasing the intracellular concentration of at least on fluorescent marker molecule selected from the group consisting of Fluorescein, Fluorescein di($\beta$-D-glucuronide), NileRed, and CY5 in Coliform bacteria in a method for rapid detection and quantification of live and recovered Coliform bacteria in a sample, comprising steps of:

    a. providing a detection cocktail comprising

        i. a nutrient medium comprising at least one of a mammalian host-derived fraction, for accelerated selective growing and multiplying of said microorganism;
        ii. at least one fluorescent marker molecule for detection of intracellular metabolism by a sensor selected from the group consisting of Fluorescein, Fluorescein di(P-D-glucuronide), NileRed, and CY5;
        iii. at least one metabolic activator selected from the group consisting of X-glucuronide, B-galactose and D-Glucose for specifically increasing metabolism of said Coliform bacteria and increasing intracellular concentration of said fluorescent marker molecule into said Coliform bacteria;

    b. contacting said sample with said detection cocktail;
    wherein said detection is microscopic detection and said quantification is by steps of:

        i. measuring gray levels of fluorescent intensity of said marker molecules by said sensor, said gray levels correlated to metabolism level of said live and recovered Coliform bacteria,
        ii. determining said gray level to a predetermined threshold of metabolism;

    further correlating said gray levels above said predetermined threshold with quantity of high metabolic-active said Coliform bacteria in said sample.

Reference is now made to an embodiment of the present invention disclosing the use mentioned above, wherein said sample comprising at least one of fluid, water, food, beverage, blood, a solution, a pharmaceutical preparation, a mammalian tissue, air, soil, surface, and any combination thereof.

Reference is now made to an embodiment of the present invention disclosing the use mentioned above, wherein host -derived fraction comprises host tissue, host tissue extract, host growth factor, and any combination thereof.

Reference is now made to an embodiment of the present invention disclosing the use mentioned above, wherein host tissue is selected from a group comprising somatic tissue, neuronal tissue, digestive tract tissue, skin, epithelial tissue, connective tissue, muscular tissue, adipose tissue, areolar tissue, bone tissue, cartilage tissue, lymphatic tissue, muscular tissue, fibrous tissue, urinary tract tissue, lymphatic tissue, liver tissue, blood serum, fetal blood serum, cerebrospinal fluid, urine, saliva, sweat, breast milk, and any combination thereof.

Reference is now made to an embodiment of the present invention disclosing the use mentioned above, wherein metabolic activator Beta-galactoside for Total *Coliform* bacteria.

Reference is now made to an embodiment of the present invention disclosing the use mentioned above, wherein said filtering further comprising a step of filtering via bacterial filter of arrange of 0.2 to 0.6 $\mu$m pore.

Reference is now made to an embodiment of the present invention disclosing the use mentioned above, wherein time period for said detection of a specific organism in a sample is less than 24 hours.

Reference is now made to an embodiment of the present invention disclosing the use mentioned above, wherein said method further comprising steps of increasing the number of recovered microorganism is said sample.

Reference is now made to an embodiment of the present invention disclosing the use mentioned above, wherein number of said specific microorganism in said sample is equal or higher than the number of Colony Forming Units of said specific organism detected by conventional Hetero-Plate Count.

Reference is now made to an embodiment of the present invention disclosing the use mentioned above, wherein said nutrient medium, BcS-EC growth medium, for selective said growing of *Coli-fonning* bacteria comprises a mixture of Peptone water, McConkey Broth, DMEM, Fetal Calf Serum, 4-Nitrophenyl $\beta$-D-glucuronide, Galactose and glucose.

Disclosed herein, but not claimed is a composition for rapid detection of the number of live and recovered specific microorganism in a sample, comprising of a detection cocktail configured to contact said sample, said cocktail comprises:

a. a nutrient medium comprising host-derived fraction, for selective growing and multiplying of said microorganism;

b. at least one fluorescent marker molecule, for detection by a sensor; and

c. at least one metabolic activator for specifically increasing metabolism of said specific microorganism and increasing internalization of said fluorescent marker molecule into said specific microorganism;

wherein said detection cocktail , when in contact with a sample containing live predetermined microorganisms is a source of fluorescence detectable by a sensor sufficient to provide gray levels of said fluorescent marker molecules; said gray levels above predetermined threshold correlated with quantity of recovered high metabolic-active said microorganism in said sample.

Disclosed herein, but not claimed is a system useful for rapid detection and quantification of specific live and recovered microorganisms in a sample, said system comprises of:

a. an *in vitro* contact module configured to contact said sample with a detection cocktail, said cocktail comprises:

i. a nutrient medium comprising at least one of host-derived fraction, for accelerated selective growing and multiplying of said microorganism;

ii. at least one fluorescent marker molecule for detection of intracellular metabolism by a sensor

iii. at least one metabolic activator for specifically increasing metabolism of said specific microorganism and increasing concentration of said fluorescent marker molecule into said specific microorganism;

b. a module for recording data on the outcome of said in vitro contacting wherein said module comprises :

i. a sensor for measuring gray levels of fluorescent intensity of said marker molecules; and

ii. a module for correlating said detected gray levels to metabolism of said specific microorganism, determining said gray level to a predetermined threshold of metabolism; and further correlating said gray levels above said predetermined threshold with quantity of recovered high metabolic-active said microorganism in said sample.

DETAILED DESCRIPTION OF PREFERRED EMBODIMENTS

**General**

The current invention relates to a method for fast detection and identifying of recoverable microorganisms using combined microscopic detection of enhanced microbial metabolism by measuring of intracellular concentration of metabolism marker molecules as well as labeled non metabolic specific markers, preceded by enhanced microorganism growth using host-driven tissues or factors.

**Recovery of stressed microorganisms**

Microorganisms present in processed food, water or the environment may be injured and hence more exacting in their growth requirements. Such organisms may be difficult to detect because they fail to grow on the selective media normally used in their isolation. Failing in bacteria isolation is also a known phenomenon in a significant number of infections in human patients. Infections without an isolated causative agent are particularly frequent in some human body districts. Furthermore, a significant number of bacterial infection cases are not associated with the isolation of the causative agent when standard microbiological methods, based on isolation of bacteria in culture media, are applied. This inadequate bacterial isolation might be due to the involvement of microorganisms hardly growing or non-recoverable in culture media.

An appreciation of the nature of sub-lethal injury and its repair by recovering microorganisms, is therefore important in detecting and enumerating microbes.

**Recoverable microorganisms:**

Viable microorganisms include forms of non-recoverable and recoverable microorganisms. Recoverable microorganisms are of the highest interest and most tested in conventional microbial tests of water, food, beverages, medicine etc. To date, the only certified identifying and quantification method for this type of the tests is conventional count of Colony forming units (CFU).

The current invention is based on the current finding that metabolism rate is much higher in recoverable microorganisms when recovering and that metabolic processes are activated by inducers specific for microbial type. More specifically, metabolism's marker compounds' dyes increase their concentration in recoverable cells with increase of metabolism.

The method is usable to practically detect any recoverable microorganism by microscopic detection of specifically activated metabolism, without the need to wait for grown of colony (HPC method). Based on this finding, current invention discloses an innovative technology to identify recoverable microorganisms in a short time. The technology combines identifying of the type of microbial cells by detecting metabolism rate, once said metabolism rate has been increased by specific inducers (metabolic activators). The metabolism rate and the intracellular concentration level of marker compounds are a function of cell activity, which is very high in recovered cells.

Additionally, quantification of intracellular concentration of marker molecules is usable to differentiate recovered (very active) from inactive ones.

Using the specific dye for the marker molecules it is possible to detect and quantify the rate and level of intracellular concentration of said molecules, in order to define, specify and count recovered microorganisms in tested sample as shown in **Fig. 1.**

### *Coliform* bacteria

*Coliform* **bacteria** refer hereinafter to rod-shaped Gram-negative non-spore forming and motile or non-motile bacteria which ferment lactose with the production of acid and gas when incubated at 35-37°C. They are a commonly used indicator of sanitary quality of foods and water. *Coliforms* are found in the aquatic environment, in soil and on vegetation; they are universally present in large numbers in the feces of warm-blooded animals. While coliforms themselves are not normally causes of serious illness, their presence is used to indicate that other pathogenic organisms of fecal origin may be present. Such pathogens include disease-causing bacteria, viruses, or protozoa and many multicellular parasites.

### CFU

Colony forming units, usually abbreviated as CFU, refer hereinafter to individual colonies of bacteria, yeast or mold. A colony of bacteria or yeast refers to a mass of individual cells of same organism, growing together. For molds, a colony is a group of hyphae (filaments) of the same mold growing together. Colony forming units are used as a measure of the number of microorganisms present in or on surface of a sample. Colony forming units may be reported as CFU per unit weight, CFU per unit area, or CFU per unit volume depending on the type of sample tested. To determine the number of colony forming units, a sample is prepared and spread or poured uniformly on a surface of an agar plate and then incubated at some suitable temperature for a number of days. The colonies that form are counted. CFU is not a measure for individual cells or spores as a colony may be formed from a single or a mass of cells or spores.

### HPC- heterotrophic plate count

The heterotrophic plate count (HPC), formerly known as the standard plate count, is a procedure for estimating the number of live heterotrophic bacteria in water. This test provides useful information about water quality and supporting data on the significance of *coliform* test results.

The HPC is useful, *inter alia,* in judging the efficiency of various treatment processes for drinking water, swimming pools, as well as for checking the quality of finished water in a distribution system. Also for applications where boilers and cooling towers are present. Heterotrophs refer hereinafter to microorganisms that require organic carbon for growth. These include bacteria, yeasts and molds. A variety of simple culture-based tests that are intended to recover a wide range of microorganisms from water are collectively referred to "heterotrophic plate count" or "HPC test" procedures.

### Gray levels

Gray level measurements are performed in order to o statistically compare staining or fluorescence intensity for a particular marker between treatments or groups. Gray levels are used hereinafter to quantify the intensity of fluorescence intensity.

The current invention relates to a methodology to identify and quantify recoverable microorganisms in a short time, based on microscopic detection of cellular metabolism within said microorganisms.

More detailed, the current invention relates to a method for a rapid and high-yield detection of a specific microorganism in a sample, comprising mixing the sample with a unique detection cocktail, and determining the number of specific microorganisms in said sample by measuring gray levels of fluorescently marked intracellular indicators of microbial metabolism and fluorescently conjugated specific non-metabolic marker molecules in said mixture; said gray levels correlating with quantity of recovered high metabolic-active said microorganism in said sample.

Said cocktail comprises: (i) a nutrient medium comprising host-derived fraction containing cell growth activators

6

from said host organism, for accelerated selective growing and multiplying of said microorganism;(ii) a fluorescently-labelled specific markers of the tested microbe specific for recognizing said microorganism; and (iii) a metabolic activator for increasing intra-cellular metabolism and at least one a fluorescently detectable marker factor that can be indicator of the said metabolism.

EXAMPLES

The following samples describe the methods and apparatus useful in practising the invention and summarize the results of the experimental work for developing a recoverable microorganism quantification method based on detection of the rate of the cellular metabolism in specifically recovered microbes, compared to the metabolism in non-activated microbes.

In addition, the document describes process of testing system design and validation.

**EXAMPLE 1:**

**Methodology for fast identifying and quantification of recoverable microorganisms using microscopic detection of cellular metabolism.**

Viable microorganisms include forms of non-recoverable and recoverable microorganisms. Recoverable microorganisms are of the highest interest and most tested in conventional microbial tests of water, food, beverages, medicine etc. To date, the only certified identifying and quantification method for this type of the tests is conventional count of Colony forming units (CFU). Microbial recovery phenomenon occurs in heterotrophic plate count (HPC) testing, when microbes reproduce multiply using media containing recovery/growth activating specific and general nutrients.

Increased metabolism phenomenon is characteristic of active cells only and increases according to cell metabolism level. Quantification of intracellular concentration of marker molecules is used to differentiate recovered (very active) from inactive ones. As part of increased metabolism, cell membrane parts, membrane receptors, specific effectors and extracellular liquids are internalized and concentrate inside the cell. The level of intracellular concentration of indicators of intracellular metabolism is a function of cell activity, which is very high in recovered cells.

Using the marker molecules' specific dye it is possible to detect and quantify the rate and level of the said indicators intracellular concentration, in order to define, specify and count recovered microorganisms in tested sample as shown in the diagram below (as presented in **Fig.1)** .

**Fig. 1** depicts intracellular concentration of marker molecule of cellular metabolism as a result of the influence of metabolism activators

Illustrations showing the effect of bacterial recovery or metabolism stimulators/activators on dyes concentrations of marker molecules in various cells: Marker molecules dyes increase their concentration (Large white ovals) in recoverable cells with increased metabolism (B). The concentration is low (Small white ovals) in non-recoverable cells (A; B and C): In non- growth and anabiotic microorganisms which have low level of metabolism, the dyes remain confined to the extracellular space. Then recovered cells that additionally labeled with markers (Pattern ovals) for species identification by double fluorescent staining and imaging (C)

**Methodology Development**

The proposed invention comprises a method based on detection of cellular metabolic rate to determine the type and number of recovered microbes in a media. The detection results of the proposed invention were further to those obtained with conventional CFU methods further revealed an equivalence between those methods, allowing the proposed invention to be used as a CFU counter.

The method was developed and validated to use for analysis of Total microbial count, Total *Coliform* bacteria and *Ps. aeruginosa* in different testing media. All tests were performed using standard microscopic, imaging and conventional microbiology lab equipment.

The main principles of methodology development are as following:

a. Determination of nutrients cocktail composition for optimal recovery of tested microbial type that contains among other things cell growth factors from host tissue
b. Determination of effectors which specifically stimulate cell metabolism in tested microbes
c. Determination of fluorescent marker molecules for intracellular metabolism in the said microbial cells
d. Development of method for preparing the tested media for microscopic examination
e. Determination of incubation conditions (minimal incubation time and temperature) for tested media in nutrient cocktail in order to allow microbes' recovery and microscopic examination in required resolution

f. Determination of conditions (time) for cell sample staining with labeled marker molecules in presence of the metabolism effectors/activators.

g. Determination of conditions for microscopic examination

h. Determination image definition for tested recovered microbes

i. Plotting of functions for correlation between microscopic cell counts and HPC counts regarding to specific testing application

**Samples:**

Samples were collected of:

a. Sludge from municipal wastewater treatment plant

b. Pasteurized milk (3%) fat

c. Freshly squeezed orange juice.

Sampling was performed at three different days or of three production batches in triplicates from each source (total of 27 samples for each testing media). Wastewater sludge was tested for total *Coliform* bacterial, pasteurized milk for *Ps.aeruginosa* and orange juice for Total bacterial contamination.

The specific recovered bacteria count was carried out in these samples. These samples were tested in parallel by conventional HPC methods relevant for the tested bacteria. Data from all analyses was used to determine the relationship between the proposed invention's procedures for cell metabolism- based recoverable microbe's determination to the number of recovered microbes of HPC methods in tested media. The correlation analysis revealed a value of $(R^2) = 0.95$ comparing proposed invention to the conventional method.

**Tested media preparation**

Wastewater sludge was diluted by iso-normal PBS in concentrations 1:10, 1:100 and 1:1000 w/v. One ml of each solution was added to 9 ml of standard MacConkey Broth (Mb) growing media and incubated using shaking incubator et 35° C for 1hr. Within incubation the solutions were filtered via 2.0 μ and 0.1 ml of the filtrate was transferred via 0.4 μm membranes.

Pasteurized milk was contaminated with calibrated *Ps.aeruginosa* culture in concentrations of 1, 10 and 100 CFU/ml. Then, 10 ml of each contaminated milk samples were added to 90 ml of cocktail contained Pseudomonas Selective Broth (CB) and Tween 40 (2%v/v) and incubated et 25° C for 7 hr. Within incubation 10 ml of the solutions were treated with HCl (pH 4.0) for 5 min, filtered via 2.0 μ and 0.1 ml of the filtrate was transferred via 0.4 μm membranes. The membranes were washed additionally by iso-normal PBS.

Orange juice was contaminated with calibrated Total bacterial culture mix in concentrations of 1, 10 and 100 CFU/ml. Then 10 ml of each contaminated juice samples were added to 90 ml of NB and incubated using shaking incubator et 25° C for 8 hr. Within incubation the solutions were filtered via 2.0 μ and 0.1 ml of the filtrate was transferred via 0.4 μm membranes.

**Recovered bacteria staining and imaging.**

Following preparations, 0.4 μm microbial membranes were treated by working solutions of CY3 membrane dyes in PBS with additions of cell metabolic activators:

a. Asparagine for *Ps.aeruginosa*

b. Beta-galactoside for Total *Coliform* bacteria

c. NB medium for Total bacteria count

in standard concentrations. Each membrane was stained at room temperature for 6 min., washed by PBS and imaged.

Imaging was done using an Axioplan 100 microscope, 0.5NA Plan Neofluor X20 objective (BP 450-490 excitation filter (Excitation: 450-490 nm; Beam splitter: FT 510 nm; Emission: 515-555 nm; Karl Zeiss). Preparations were imaged by standard Axiocam 506 mono 5 megapixel CCD. Data collection, image processing and analyzing were performed by ImagePro+ software.

**Recoverable bacteria microscopic characteristics finding:**

a. Each type of the tested bacteria were prepared by two different procedures:

b. Following proliferation bacterial suspension were dispensed for two same portions. The first portion was proliferated by our procedures as described previously. The second portion was shocked metabolically by incubation in iso-normal saline at 25°C for 72 hr. The first portion was then stained in presence and the second portion in the absence of cell metbolic activators (see previous paragraphs).

c. The recovery activated and shocked cultures were microscopically observed and imaged.

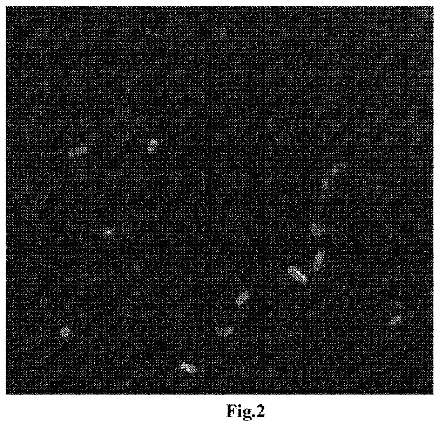

d. Morphology and light intensity characteristics of microbes in each culture were measured and analyzed. Morphology characteristics of bacteria were similar in activated and shocked cultures regarding to specific bacterial type **(Fig. 2).**

e. Along with this, fluorescent intensity of activated bacteria was found to be significantly higher.

The following observations were made during imaging by current imaging setup:

a. The fluorescent intensity of stained bacteria was in the range of 25-225 gray levels (on a scale of 0 to 255)

b. Shocked bacteria had a signal of 25 - 55 gray levels

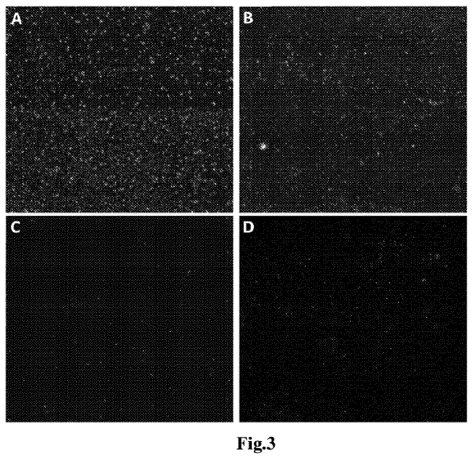

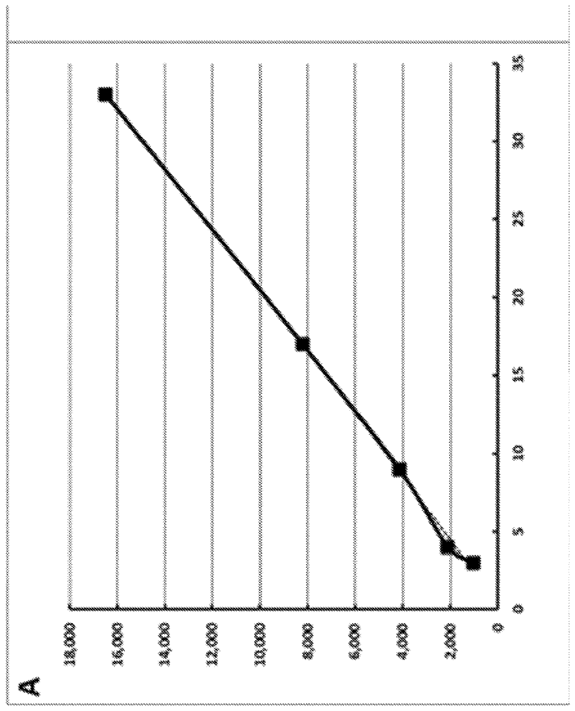

c. Cellular metabolism- activated microorganisms had a signal of 85 - 225 gray levels **(Fig. 3).**

Therefore, a difference of about 30 gray levels was measured between the recovered (cellular metabolism-stimulated) and non-recovered microorganisms.

**Fig. 3** presents intracellular concentrating of marker molecules in recovered or metabolism-activated Total *Coliforms* (A) and *Ps. aeruginosa* (B) cultures. Significantly low concentration of marker molecules was observed in metabolically shocked Coliforms (C) and in *A. hydrophilia* treated by unspecific activators (D).

**Characteristics used for recovered microbial cells identifying in tested media.**

*Correlations of proposed invention to conventional (HPC) counts:*

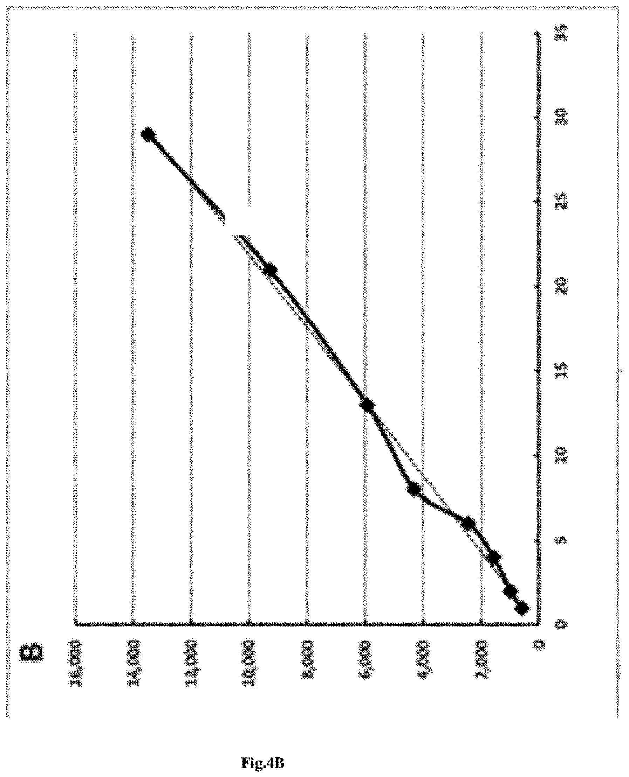

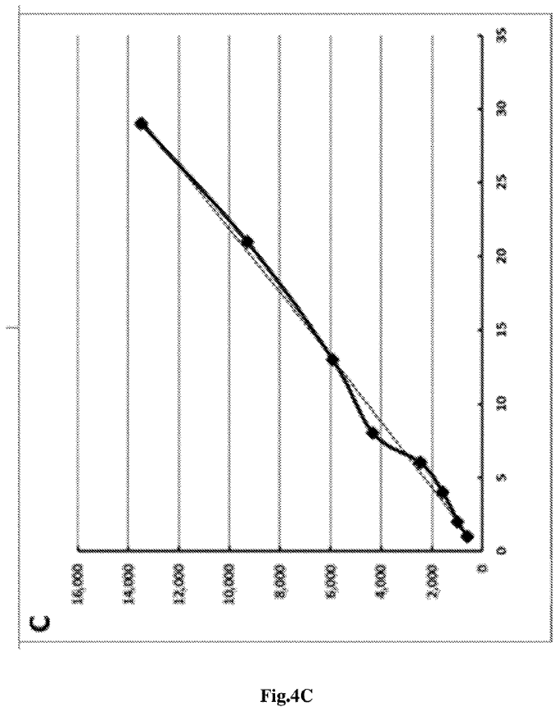

Samples of Total *Coliform* bacteria isolated from wastewater, Total bacteria mix obtained from orange juice and standard *Ps.aeruginosa* culture were diluted by PBS to concentrations of 1 and 10 CFU/ml. One ml of each diluted sample was added to selective bacteria recovery activating media and incubated as described previously. Then the solutions were treated, stained and imaged according to proposed invention, each for 0.5 hr. Twenty percent (20%) of each preparation was imaged. In parallel, 1ml of each solution was tested using conventional HPC methodology. All tests were prepared in triplicates. The results obtained from both methods were averaged, normalized to 1ml of starting microbial suspensions, compared (See **Table 1)** and plotted. Correlation equations for each culture are shown in graphs (See **Fig. 4).**

**Fig. 4** presents the definition of initial sample contamination.

Bacterial counts which were performed by Heterotrophic plate count method (Normalized to 1ml volume, Axis Y) and by the new method (Raw count, Axis X) for *E. coli* (A); *Ps. aeruginosa* (B) and Total heterotrophic bacteria (C) are plotted to getting trendlines and equations. This process revealed formulas for calculating of initial contamination counts.

The initial contamination levels for tested media were calculated as following:

$$\mathbf{N_i = F_c(x)/2^{(T_s/T_c)}}$$

Where: Ni - Initial contamination level;

Fc - Correlation equation (experimental data);

x- Count by New developed method [RMUTs - Sampling (incubation) time [min.];Tc - Cell cycle (dividing) period [min.] (experimental data).

Examples:

Coliforming bacteria:

$$\mathbf{N_i = (0.8076x^2 + 477.42x - 135.61)/(2^{(T_s/30)})}$$

Poemginosa:

$$Ni = (0.7353x^2 + 436.39x + 108.41)/(2^{(Ts/45)})$$

Total bacteria:

$$Ni = (-2.5276x^2 + 464.7x - 420.33)/(2^{(Ts/50)})$$

### A. Coliforming bacteria

| Sample | Initiated contamination 1CFU/ml | | | | Initiated contamination 10CFU/ml | | | |
|---|---|---|---|---|---|---|---|---|
| | HPC count | | New method | | HPC count | | New method | |
| | Count | Norm. | Count | Norm. | Count | Norm. | Count | Norm. |
| [hr.] | [CFU] | [CFU/ml] | [RMU] | [RMU/ml] | [CFU] | [CFU/ml] | [RMU] | [RMU/ml] |
| 0.0 | ND | NP | ND | NP | 0 | NP | ND | NP |
| 2.0 | 2 | 20 | ND | NP | 14 | 140 | ND | NP |
| 3.0 | 7 | 70 | ND | NP | 66 | 660 | ND | NP |
| 4.0 | 26 | 260 | ND | NP | 241 | 2,410 | 5 | 2,500 |
| 5.0 | 104 | 1,040 | 3 | 1,500 | 885 | 8,850 | 17 | 8,500 |
| 5.5 | 212 | 2,120 | 4 | 2,000 | 1,542 | 15,420 | 34 | 17,000 |
| 6.0 | 415 | 4,150 | 9 | 4,500 | TNTC | NP | 68 | 34,000 |
| 6.5 | 822 | 8,220 | 17 | 8,500 | TNTC | NP | 114 | 57,000 |
| 7.0 | 1,650 | 16,500 | 33 | 16,500 | TNTC | NP | 242 | 121,000 |
| 7.5 | TNTC | TNTC | 65 | 32,500 | TNTC | NP | 511 | 255,500 |
| 8.5 | TNTC | TNTC | 262 | 131,000 | TNTC | NP | 2,236 | 1,118,000 |
| 9.5 | TNTC | TNTC | 1,158 | 579,000 | TNTC | NP | TNTC | NP |
| 12.0 | TNTC | TNTC | TNTC | NP | TNTC | NP | TNTC | NP |

### B. Ps.aeruginosa

| Sample | Initiated contamination 1CFU/ml | | | | Initiated contamination 10CFU/ml | | | |
|---|---|---|---|---|---|---|---|---|
| | HPC count | | New method | | HPC count | | New method | |
| | Count | Norm. | Count | Norm. | Count | Norm. | Count | Norm. |
| [hr.] | [CFU] | [CFU/ml] | [RMU] | [RMU/ml] | [CFU] | [CFU/ml] | [RMU] | [RMU/ml] |
| 0.0 | ND | NP | ND | NP | ND | NP | ND | NP |
| 3.0 | 2 | 20 | ND | NP | 15 | 150 | ND | NP |
| 4.0 | 3 | 30 | ND | NP | 36 | 360 | ND | NP |
| 6.0 | 24 | 240 | ND | NP | 246 | 2,460 | 5 | 2,560 |
| 7.0 | 61 | 610 | 1 | 500 | 621 | 6,210 | 13 | 6,451 |
| 7.5 | 99 | 990 | 2 | 1,000 | 884 | 8,840 | 22 | 11,000 |
| 8.0 | 158 | 1,580 | 4 | 2,000 | 1,488 | 14,880 | 33 | 16,255 |

(continued)

| Sample | Initiated contamination 1CFU/ml | | | | Initiated contamination 10CFU/ml | | | |
|---|---|---|---|---|---|---|---|---|
| | HPC count | | New method | | HPC count | | New method | |
| | Count | Norm. | Count | Norm. | Count | Norm. | Count | Norm. |
| **8.5** | 245 | 2,450 | 6 | 3,000 | 2,287 | 22,870 | 58 | 29,000 |
| **9.0** | 432 | 4,320 | 8 | 4,000 | TNTC | NP | 82 | 40,960 |
| **9.5** | 593 | 5,930 | 13 | 6,500 | TNTC | NP | 141 | 70,500 |
| **10.0** | 930 | 9,300 | 21 | 10,500 | TNTC | NP | 206 | 103,213 |
| **10.5** | 1,350 | 13,500 | 29 | 14,500 | TNTC | NP | 344 | 172,000 |
| **12.0** | TNTC | NP | 128 | 64,000 | TNTC | NP | 1,311 | 655,360 |

*C.* **Total bacteria**

Table 1 Microbial counts of New developed method (RMU- recovered microbial count) correlated to HPC method (CFU-colony forming units). Results for Coliforming bacteria (A), Ps.aeruginosa (B) and Total bacteria count (C) are presented in forms of raw and normalized to 1 ml counts.

| Sample | Initiated contamination 1CFU/ml | | | | Initiated contamination 10CFU/ml | | | |
|---|---|---|---|---|---|---|---|---|
| | HPC count | | New method | | HPC count | | New method | |
| | Count | Norm. | Count | Norm. | Count | Norm. | Count | Norm. |
| [hr.] | [CFU] | [CFU/ml] | [RMU] | [RMU/ml] | [CFU] | [CFU/ml] | [RMU] | [RMU/ml] |
| **0.0** | ND | NP | ND | NP | ND | NP | ND | NP |
| **3.5** | 2 | 20 | ND | NP | 16 | 160 | ND | NP |
| **5.5** | 8 | 80 | ND | NP | 89 | 890 | 2 | 1,000 |
| **7.5** | 49 | 490 | ND | NP | 421 | 4,210 | 12 | 6,000 |
| **8.0** | 68 | 680 | 2 | 1,000 | 687 | 6,870 | 15 | 7,500 |
| **8.5** | 102 | 1,020 | 3 | 1,500 | 1,016 | 10,160 | 26 | 13,000 |
| **9.0** | 142 | 1,420 | 5 | 2,500 | 1652 | 16,520 | 38 | 19,000 |
| **9.5** | 231 | 2,310 | 7 | 3,500 | TNTC | NP | 49 | 24,500 |
| **10.0** | 378 | 3,780 | 8 | 4,000 | TNTC | NP | 92 | 46,000 |
| **10.5** | 544 | 5,440 | 14 | 7,000 | TNTC | NP | 142 | 71,000 |
| **11.0** | 870 | 8,700 | 22 | 11,000 | TNTC | NP | 201 | 100,500 |
| **11.5** | 1120 | 11,200 | 30 | 15,000 | TNTC | NP | 312 | 156,000 |
| **12.0** | TNTC | NP | 48 | 24,000 | TNTC | NP | 511 | 255,500 |

**Comparison of wild diluted samples with contaminated samples**

Total microbial mix from orange juice was cultured in broth medium for 72 hr at 25°C. The medium was removed by centrifugation and microbial pellet was used for juice contamination of 1, 10 and 100 CFU/ml. Pasteurized milk was contaminated with standard *Ps.aeruginosa* by the same procedure. Wastewater sludge was taken as is and diluted as described previously.

Contaminated and wild diluted samples were tested in triplicates by conventional (HPC) and the new method of the proposed invention, simultaneously. The testing was repeated in three different experimental days for each kind of the test. Results of microscopic observations were processed for recovered bacteria counts as described previously.

The counts of both kinds of tests were averaged regarding to experimental day, normalized for 1ml of initial sample and correlated. Summary of the testing results is presented in **Table 2.**

| *Coliforming* bacteria count in Wastewater sludge |
|---|

| Experimental day | Initial dillution | HPC count | New method | | Correlation |
|---|---|---|---|---|---|
| | | Norm. | Raw | Norm. | |
| | | [CFU/gr] | [RMUl] | [RMU/gr] | [%] |
| **1** | 1:10 | 1,210 | 11 | 1,303 | 92 |
| | 1:100 | 1,390 | 13 | 1,552 | 88 |
| | 1:1000 | 1,320 | 12 | 1,427 | 92 |
| **2** | 1:10 | 2,040 | 17 | 2,053 | 99 |
| | 1:100 | 1,960 | 18 | 2,180 | 89 |
| | 1:1000 | 2,140 | 19 | 2,307 | 92 |
| **3** | 1:10 | 1,540 | 14 | 1,677 | 91 |
| | 1:100 | 1,520 | 14 | 1,677 | 90 |
| | 1:1000 | 1,730 | 15 | 1,802 | 96 |

### *Ps.aeruginosa* bacteria count in Milk

| Experimental day | Initial contamination | HPC count | New method | | Correlation |
|---|---|---|---|---|---|
| | | Norm. | Raw | Norm. | |
| | [CFU/ml] | [CFU/ml] | [RMUl] | [RMU/ml] | [%] |
| **1** | 1 | ND | 3 | 2 | NC |
| | 10 | 8 | 13 | 9 | 88 |
| | 100 | 97 | 132 | 109 | 87 |
| **2** | 1 | 2 | 4 | 3 | 55 |
| | 10 | 26 | 42 | 31 | 82 |
| | 100 | 165 | 195 | 175 | 94 |
| **3** | 1 | 3 | 5 | 4 | 81 |
| | 10 | 31 | 48 | 35 | 86 |
| | 100 | 186 | 211 | 194 | 96 |

### *Total* bacteria count in Orange juice

| Experimental day | Initial contamination | HPC count | New method | | Correlation |
|---|---|---|---|---|---|

| | Norm. | Raw | | Norm. | |
|---|---|---|---|---|---|
| | [CFU/ml] | [CFU/ml] | [RMUl] | [RMU/ml] | [%] |
| **1** | 1 | 2 | 5 | 2 | 100 |
| | 10 | 12 | 19 | 13 | 92 |
| | 100 | 99 | 129 | 106 | 93 |
| **2** | 1 | 1 | 2 | 2 | 47 |
| | 10 | 8 | 14 | 10 | 75 |
| | 100 | 88 | 115 | 93 | 94 |
| **3** | 1 | ND | 1 | 1 | NC |
| | 10 | 9 | 14 | 10 | 90 |
| | 100 | 87 | 111 | 89 | 97 |

**Table 2.** Testing results of New developed method (RMU) and HPC method (CFU). *Coliforming* bacteria (A) count in wastewater sludge, *Ps.aeruginosa* count in milk (B) and Total bacteria count in orange Juice (C) are presented and normalized to 1 ml of the sample .The correlations per each test were

The results were obtained:

Conventional method:

24 hr. for *Coliform* bacteria
72 hr. for *Ps.aeruginosa*
96 hr. for Total bacterial count.

New method:

1 hr. for *Coliform* bacteria
7 hr. for *Ps.aeruginosa*
8 hr. for Total bacterial count.

The testing time needed for results obtaining by the new method is depended and inversely proportional to starting contamination of tested media and microbial cell cycle (dividing time).

**Fig. 5** depicts the correlation of microbial counts between the tested and conventional methods. In most cases the correlation between the new and conventional method is defined as about 90% (see **Fig. 5).** The correlations were tested by counts of *E.coli* ( rhombus), *Ps.aeruginosa* (square) and Total heterotrophic bacterial count (triangle).

X and Y axis represent No. of experimental series [hr.] and ration of numerical correlation ratio [%] respectively.

In most cases the correlation between the new and conventional method is defined as about 90% (see **Fig. 5)** which presents media testing correlation. **Fig. 5** presents correlation between the tested methods and the sample number).

**Summary of EXAMPLE 1**

The results of example 1 show the validity of the new methodology of the proposed invention, and the equivalence of said new methodology to CFU counting method, for example samples of Total, *Coliform* and *Ps.aeruginosa* recoverable bacteria count in wastewater sludge, juices and milk.

Those three types' bacterial contaminations are of utmost importance in food, beverages and environmental microbiology.

Recoverable count using the new methodology will usually provide larger numbers than CFU tested conventionally: Conventional HPC count is an indirect test based on culture of microbes. The cultivability of a microorganism depends on properties of growing media and incubation conditions.

According to this, it is expected that direct new testing system detect more recovered cells.

Comparisons of conventional HPC and new methodology for recoverable microorganisms quantification

Characteristics of both testing methods are summarized below

Table 3: Comparison characteristics of conventional method to current invention

| Criteria | New method | Conventional HPC |
|---|---|---|
| Working time (sampling and transportation not included) | 5-7 min. | 10 min. |
| Time to receive the results | 1-8 hr. | 24 hr. - 21 days |
| Authorized personnel required | Yes | Yes |
| Cost per test for performer | 1.5-2.0 $ | 1.0 $ |
| Cost per test for customer | 4 - 6 $ | 8 $ |
| Required equipment cost | 30 - 40 k$ | 25 - 35 k$ |
| Accuracy | $\pm$ 5 % | $\pm$ 35 % |
| Ability of total automation | Yes | No |
| Sensitivity | High | Medium |
| Time required for periodical validation | 3 months | 3 months |
| Time required for new application validation | 3 month | 2 weeks |
| Special working space required | Yes | Yes |
| **Ability to proceed close to sampling or production area** | Yes | No |

Fig. 6 depicts that media containing growth activators enables commencing of bacterial detection 20-80% sooner than standard growth media. Solid horizontal line specifies levels of specific bacteria contamination that are sufficient to reliable microbial detection and enumeration by the new technology.

The lines on the graph are the growth curves of *E.coli* in the newly invented (square) and standard peptone water (triangle) or MacConkey (rhombus) growing broths. The increase in growth rate (marked by oval shapes) is verified for initial concentrations of 10 CFU/ml. (solid lines) and 100 CFU/ml (dotted line).

X and Y axis represent incubation time [hr.] and bacterial concentration [CFU/ml] respectively.

**EXAMPLE 2:**

**Preparation of microbial metabolism activation/staining cocktail**

The staining cocktail contains three main components. These components are used at the same time or separately, depending on the tested bacteria and/or tested material.

*Specific or non-specific activation of the cellular metabolism:*

This reagent contains a substance that in selective or non-selective manner increase intracellular metabolism of the tested microbe. The substances that are usable are specific or no-specific sugars, proteins, effectors of membrane receptors, specific substrates of intracellular enzymatic reactions.

This reagent contains

**Detection of high metabolism**: This reagent contains a fluorescent detectable substance that is absorbed by a microbial cell and accumulates in it due to the rise in cellular metabolism. Substances that participate in intracellular metabolic reactions that have light /fluorescent properties are suitable.

**Specific identification of microbes**: This reagent contains a fluorescent detectable substance that specifically and accurately identify type of microbial species.

The reagents are chosen of:

- fluorescent conjugated antibodies
- metabolized carbonates
- specific DNA/RNA sequences
- fluorescent products of specific intracellular reactions

Note- In some cases, the use of the same substances meet both purposes.

Using specific dyes, it is possible to detect and quantify the rate and level of internalization of the selected compounds to count and identify recoverable microorganisms in a tested sample.

The following are non -limiting examples for cocktails preparations.

a. A cocktail for *Ps.aeruginosa* testing:
b. Bactosense Mediums for Pseudomonas Enhancement Growth.
c. Cetrimide (10 mg/l)-a metabolism activator.
d. Fucidin(10 mg/l)-a metabolism activator.
e. Fluorescein, a selective marker of metabolism naturally produced in metabolically active *Ps.aeruginosa* cells.
f. Anti-Pseudomonas aeruginosa monoclonal antibody (for example ab35835) (1:50), an identification marker.

**2. A cocktail for E.coli testing:**

a. Bactosense Mediums for *Coliform* bacteria Enhancement grows
b. X-glucuronide (0.075 g/1), a metabolism activator.
c. B-galactose (1.0 g/1), a metabolism activator.
d. Fluorescein di($\beta$-D-glucuronide) (5 mg/), a selective metabolism marker.
e. Anti-E.coli FITC -(for example AB30522) (1:50), an identification marker.

**3. A cocktail for *Coliform* bacteria testing:**

a. Bactosense Mediums for Coliform bacteria Enhancement grows
b. D-Glucose (2 g/1), a metabolism activator.
c. B-galactose (1.0 g/1), a metabolism activator.
d. NileRed (5 mg/l), a general marker of metabolism; or
**e.** CY5 (1 mg/l), a general marker of metabolism.

**EXAMPLE 3:**

**Special Media for Enhancement Growth of Different Microorganisms**

1. Growth media functions:

a. Simulates host tissue/fluid environment.
b. Includes various growth factors/stimulators/triggers.
c. Controlled environmental parameters.
d. Supports enhanced growth rates of microorganisms.

e. Simulates host tissue/fluid environment

The current invention teaches that growth of pathogenic bacteria is stimulated by growth factors found in the host organism / tissue rather than those found in alternative sources. This is a logical assumption since a given pathogenic microbe will proliferate intensively in a specific type host organism / tissues, but will be harmless and /or will not proliferate in others.

Thus, it is possible to accelerate the microbial pathogen cultivation by addition of individual or mixed cell growth accelerators, which simulate host tissue/fluid environment, into the standard growth media.

This assumption was tested experimentally. The results indeed show acceleration of bacterial growth rate in ranges of 20-75%

a. Includes various growth factors/stimulators/triggers

The activator is chosen of:

   i. Mixed /complicated activators:

   Tissues and/or tissue extracts from host of the pathogen - antibody free blood serum, fetal blood serum, somatic/neuronal tissue extracts, CSF, urine and/urine tract tissue extracts, lysozyme free saliva, etc.
   Mix of cell growth factors (cytokines) from the host organisms

   ii. Isolated cytokines from the host organism /tissues.

For designing the growth media formulation, types and concentrations of the additions should be tested according to their influence on cultivation rate of the specific pathogen.

b. Design of formulation of the growth media:

   i. Select the optimal standard selection medium (usually liquid) for the specific pathogen's growth.
   ii. Select growth activator known to be strongly associated with the microorganism in the host.
   iii. Select the environment that effectively supports growth of the host cells /tissues (standard mixture).
   iv. Define concentration of the activator/s in the environment (activating mixture).
   v. Define proportions between the standard and the activation mixtures in final formulation of the medium.
   vi. Bring the content of selecting factors from the standard media up to the optimal levels.

c. Media quality test:

   i. Compare microbial growth rates using Standard and Novell designed media by HPC or Five tubes methods.
   ii. Define storage conditions and shelf life of the media by determining the microbial growth rate in it.

2. Examples for growth media

a. BcS-EC growth medium

BcS-EC is a special bacteria growth media for accelerated specific growth of *Coli-forming* bacteria.

Reagents:

For preparing of 1 L and/ or 0.5 L BcS-EC media needed the following reagents are described in the **Table 4:**

**Table 4: Composition of BcS-EC media**

| № | Reagent | Storag e | Amount | | Cat. number | Manufacturer |
|---|---------|----------|--------|--|-------------|--------------|
| | | | 500ml | 1000ml | | |
| 1 | Peptone Water | RT | 125ml | 250ml | 7365A | Neogen |
| 2 | McConkey Broth | RT | 250ml | 500ml | 8468.0 0500 | VWR Chemicals |
| 3 | DMEM (4.5g)* | +4∘C | 100ml | 200ml | 11-055-1g | Biological Ind. |
| 4 | Fetal Calf Serum (FCS)* | -20∘C | 25ml | 50ml | 04-001-1A | Biological Ind. |
| 5 | 4-Nitrophenyl β-D-glucuronide | RT | 10 mg | 5 mg | N1627 | Sigma |
| 6 | Galactose | +4∘C | 2.5g | 5 g | G0625-100G | Sigma |
| 7 | Glucose | +4∘C | 1g | 2g | D8270-100G | Sigma |
| *  *Unique additions for bacterial growth* | | | | | | |

Procedure:

i. Prepare Peptone Water, McConkey Broth and DMEM according to the manufacturer's instructions:
ii. Dissolve 13.38g DMEM High Glucose (4.5g/L) in 1L DDW with addition of 3.7 g/L Sodium Bicarbonate (without heating);
iii. Suspend 40.01 g MacConkey Broth in 1L DDW with magnet stirrer and heating;
iv. Dissolve 15g Peptone Water in 1L DDW with magnet stirrer and heating;
v. Mix together Peptone Water, MacConkey and DMEM solutions.
vi. Add FCS, Galactose and Glucose to this solution and mix well with magnet stirrer.
vii. Sterilize solution by filtration through 0.22$\mu$m membrane.
viii. Aseptically divide to aliquots of 10 and/ or 50ml into sterile plastic tubes.
ix. Store in refrigerator at + 4∘C.

b. BcS-LM

BcS-LM is a special bacteria growth media for accelerated specific growth of *Listeria.*

Reagents:

For preparing of 1 L and/or 0.5L DGD-LM media needed the following reagents are described in **Table 5**:

**Table 5: Composition of BcS-LM media**

| № | Reagent | Storag e | Amount | | Cat. number | Manufacturer |
|---|---------|----------|--------|--|-------------|--------------|
| | | | 500ml | 1000ml | | |
| 1 | Listeria Broth | RT | 375ml | 750ml | 84652.0500 | VWR Chemicals |
| 2 | DMEM (4.5g)* | +4∘C | 100ml | 200ml | 11-055-1g | Biological Ind. |
| 3 | Fetal Calf Serum (FCS)* | -20∘C | 25ml | 50ml | 04-001-1A | Biological Ind. |
| 4 | Nalidixic acid | RT | 10 mg | 20 mg | 158542 | ALDRICH |
| 5 | Cycloheximide | RT | 10 mg | 20 mg | C4859 SIGMA | SIGMA |
| 6 | Galactose | +4∘C | 2.5g | 5g | G0625-100G | Sigma |
| 7 | Glucose | +4∘C | 1g | 2g | D8270-100G | Sigma |
| *  *Unique additions for bacterial growth* | | | | | | |

**2.2 Procedure**

i. Prepare Listeria Broth according to the manufacturer's instructions:
ii. Dissolve 15g in 1L DDW with magnet stirrer;
iii. Add supplements for Listeria Broth and mix well with stirrer.
iv. Add DMEM, FCS, Galactose and Glucose. Mix well with magnet stirrer.
v. Sterilize solution by filtration through 0.22$\mu$m membrane.
vi. Aseptically divide to aliquots of 10-50ml into sterile plastic tubes.
vii. Store in refrigerator at 4∘C.

## Composition of FBS

| Component | Average | Range |
|---|---|---|
| Endotxins (ng/ml) | 0.35 | 0.01 - 10.0 |
| Glucose (mg/ml) | 1.25 | 0.85 - 1.81 |
| Protein (mg/ml) | 38 | 32 - 70 |
| Albumin (mg/ml) | 23 | 20 - 36 |
| Hemoglobine (µg/ml) | 113 | 24 - 181 |
| Bilirubin, total (µg/ml) | 4 | 3 - 11 |
| Bilirubin, direct (µg/ml) | 2 | 0 - 5 |
| Urea (µg/ml) | 160 | 140 - 200 |
| Urate (µg/ml) | 29 | 13 - 41 |
| Creatinin (µg/ml) | 31 | 16 - 43 |
| Insulin (µU/ml) | 10 | 6 - 14 |
| Cortisol (ng/ml) | 0.5 | 0.1 - 23 |
| Growth hormone (ng/ml) | 39.0 | 18.7 - 51.6 |
| Parathormone, PTH (ng/ml) | 1.72 | 0.085 - 6.18 |
| Triiodothyronine, T3 (ng/ml) | 1.2 | 0.56 - 2.23 |
| Thyroxine, T4 (ng/ml) | 0.12 | 0.08 - 0.16 |
| Thyroid-stimulating hormone, TSH (ng/ml) | 1.22 | 0.2 - 4.5 |
| Follicle-stimulating hormone, FSH (pg/ml) | 95 | 20 - 338 |
| Testosterone (pg/ml) | 400 | 210 - 990 |
| Progesterone, P4 (pg/ml) | 80 | 3 - 360 |
| Prolactin = Luteotropic hormone, LTH (pg/ml) | 176 | 20 - 500 |
| Luteinizing hormone, LH ?? (pg/ml) | 8 | 1.2 - 18 |
| Prostaglandin E (ng/ml) | 5.9 | 0.5 - 30.5 |
| Prostaglandin F (ng/ml) | 12.3 | 3.8 - 42.0 |
| Vitamine A (ng/ml) | 90 | 10 - 350 |
| Vitamine E (ng/ml) | 1.1 | 1 - 4.2 |
| Cholesterol (µg/ml) | 310 | 120 - 630 |
| Lactate-dehydrogenase, LDH (mU/ml) | 864 | 260 - 1,215 |
| Alkaline Phosphatase (mU/ml) | 255 | 110 - 352 |
| Aspartate-Aminotransferase, ASAT (mU/ml) | 130 | 20 - 200 |

## Table 6: Composition of FCS (Fetal Calf Serum)

*from Lindl, T. (2002): "Zell- und Gewebekultur". 5th ed. Spektrum Akademischer Verlag, Heidelberg*

*According to Thermo's data, 111ng/ml IGF, 12.6ng/ml TGF-beta, 37.3ng/ml FGF-2 is present in FBS. (However there are batch- to batch variations).*

**EXAMPLE 4:**

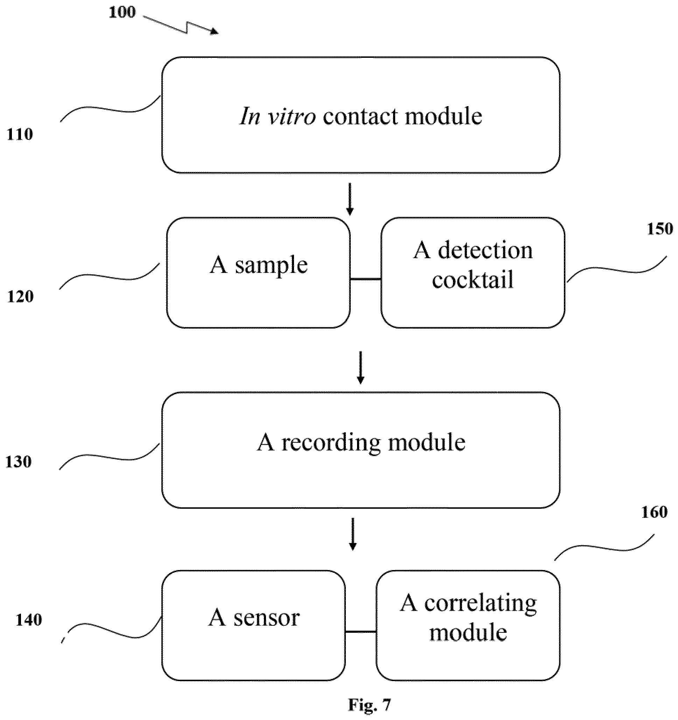

Reference is now made to **Figure 7,** illustrating a system (100) useful for rapid detection and quantification of specific live and recovered microorganisms in a sample (120) , The system comprises

a. an in vitro contact module (110) configured to contact the sample (120) with a detection cocktail, (150) , the cocktail (150) comprises:

i.a nutrient medium comprising at least one of host-derived fraction or host sourced cell growth factors, for accelerated selective growing and multiplying of the microorganism;
ii. at least one fluorescent marker molecule for detection of intracellular metabolism by a sensor
iii. at least one metabolic activator for specifically increasing metabolism of said specific microorganism and increasing concentration of the fluorescent marker molecule into said specific microorganism;

b. a module for recording data (130) on the outcome of said in vitro contacting wherein the module comprises :

i.a sensor (140) for measuring gray levels of fluorescent intensity of the marker molecules; and
ii.a module for correlating (160) said detected gray levels to metabolism of the specific microorganism, determining the gray level to a predetermined threshold of metabolism; and further correlating the gray levels which are above the predetermined threshold with quantity of recovered high metabolic-active microorganism in the sample.

**Claims**

1. Use of X-glucuronide, B-galactose and D-Glucose for increasing the intracellular concentration of at least on fluorescent marker molecule selected from the group consisting of Fluorescein, Fluorescein di($\beta$-D-glucuronide), NileRed, and CY5 in Coliform bacteria in a method for rapid detection and quantification of live and recovered Coliform bacteria in a sample, comprising steps of:

a. providing a detection cocktail comprising

i. a nutrient medium comprising at least one of a mammalian host-derived fraction, for accelerated selective growing and multiplying of said microorganism;
ii. at least one fluorescent marker molecule for detection of intracellular metabolism by a sensor selected from the group consisting of Fluorescein, Fluorescein di($\beta$-D-glucuronide), NileRed, and CY5;
iii. at least one metabolic activator selected from the group consisting of X-glucuronide, B-galactose and D-Glucose for specifically increasing metabolism of said Coliform bacteria and increasing intracellular concentration of said fluorescent marker molecule into said Coliform bacteria;

b. contacting said sample with said detection cocktail;
wherein said detection is microscopic detection and said quantification is by steps of:

i. measuring gray levels of fluorescent intensity of said marker molecules by said sensor, said gray levels correlated to metabolism level of said live and recovered Coliform bacteria,
ii. determining said gray level to a predetermined threshold of metabolism;

further correlating said gray levels above said predetermined threshold with quantity of high metabolic-active said Coliform bacteria in said sample.

2. The use of claim 1, further comprising a step of filtering said sample via bacterial filter of a range of 0.2 to 0.6 $\mu$m pore.

3. The use of claim 1, wherein a time period for said detection of said Coliform bacteria in a sample is less than 24 hours.

4. The use of claim 1, wherein:

(a) when said detection cocktail comprises Peptone water, McConkey Broth, DMEM, Fetal Calf Serum, 4-Nitrophenyl $\beta$-D-glucuronide, B-galactose and D-Glucose.

**Patentansprüche**

1. Verwendung von X-Glucuronid, B-Galactose und D-Glucose zur Erhöhung der intrazellulären Konzentration von mindestens einem fluoreszierenden Markermolekül, ausgewählt aus der Gruppe bestehend aus Fluorescein, Fluorescein di(β-D-glucuronid), NileRed und CY5 in coliformen Bakterien in einem Verfahren zur schnellen Detektion und Quantifizierung von lebenden und rückgewonnenen coliformen Bakterien in einer Probe, umfassend die Schritte:

    a. Bereitstellung eines Detektionscocktails, umfassend

        i. ein Nährmedium, das mindestens eine von einem Säugerwirt stammende Fraktion für das beschleunigte selektive Wachstum und die Vermehrung des Mikroorganismus umfasst;

        ii. mindestens ein fluoreszierendes Markermolekül zur Detektion des intrazellulären Metabolismus durch einen Sensor, ausgewählt aus der Gruppe bestehend aus Fluorescein, Fluorescein di((3-D-glucuronid), NileRed und CY5;

        iii. mindestens einen metabolischen Aktivator, ausgewählt aus der Gruppe bestehend aus X-Glucuronid, B-Galactose und D-Glucose, um den Metabolismus der coliformen Bakterien spezifisch zu steigern und die intrazelluläre Konzentration des fluoreszierenden Markermoleküls in den coliformen Bakterien zu erhöhen;

    b. Inkontaktbringen der Probe mit dem Detektionscocktail;

    wobei die Detektion eine mikroskopische Detektion ist und die Quantifizierung durch die Schritte erfolgt:

        i. Messung der Grauwerte der Fluoreszenzintensität der Markermoleküle durch den Sensor, wobei die Grauwerte mit dem Metabolismusniveau der lebenden und rückgewonnenen coliformen Bakterien korrelieren,

        ii. Bestimmung der Grauwerte in Bezug auf einen vorbestimmten Schwellenwert für den Metabolismus;

    weiterhin Korrelation der Grauwerte oberhalb des vorbestimmten Schwellenwerts mit der Menge der hochgradig metabolisch aktiven coliformen Bakterien in der Probe.

**2.** Verwendung nach Anspruch 1, ferner umfassend einen Schritt des Filtrierens der Probe über einen Bakterienfilter mit einer Porengröße im Bereich von 0,2 bis 0,6 μm.

**3.** Verwendung nach Anspruch 1, wobei die Zeitspanne für die Detektion der coliformen Bakterien in einer Probe weniger als 24 Stunden beträgt.

**4.** Verwendung nach Anspruch 1, wobei:

    (a) wenn der Detektionscocktail Peptonwasser, McConkey-Bouillon, DMEM, fötales Kälberserum, 4-Nitrophenyl-(3-D-glucuronid, B-Galactose und D-Glucose umfasst.

## Revendications

**1.** Utilisation de X-glucuronide, de β-galactose et de D-glucose pour augmenter la concentration intracellulaire d'au moins une molécule de marqueur fluorescent choisie dans l'ensemble constitué par les fluorescéine, di(β-D-glucuronide) de fluorescéine, rouge du Nil et CY5 chez des bactéries coliformes, dans une procédé de détection et quantification rapides de bactéries coliformes vivantes et récupérées au sein d'un échantillon, procédé qui comporte les étapes suivantes :

    a) fournir un cocktail de détection comprenant

        i) un milieu nutritif comprenant au moins une fraction dérivée d'un hôte mammifère, pour croissance et multiplication sélectives accélérées dudit microorganisme,

        ii) au moins une molécule de marqueur fluorescent, pour détection du métabolisme intracellulaire par un capteur, choisie dans l'ensemble constitué par les fluorescéine, di(β-D-glucuronide) de fluorescéine, rouge du Nil et CY5,

        iii) au moins un activateur du métabolisme, choisi dans l'ensemble constitué par les X-glucuronide, β-galactose et D-glucose, pour augmenter spécifiquement le métabolisme desdites bactéries coliformes et augmenter la concentration intracellulaire de ladite molécule de marqueur fluorescent chez lesdites bactéries coliformes,

b) et mettre ledit échantillon en contact avec ledit cocktail de détection ;

et dans lequel ladite détection est une détection au microscope et ladite quantification passe par les étapes suivantes :

i) mesurer les niveaux de gris de l'intensité de la fluorescence desdites molécules marqueurs au moyen dudit capteur, lesquels niveaux de gris sont corrélés au niveau du métabolisme desdites bactéries coliformes vivantes et récupérées,
ii) et déterminer ledit niveau de gris par rapport à un seuil prédéterminé du métabolisme ;

et corréler en outre lesdits niveaux de gris situés au-dessus dudit seuil prédéterminé avec la quantité, dans ledit échantillon, desdites bactéries coliformes à métabolisme hautement actif.

2. Utilisation conforme à la revendication 1, qui comprend en outre une étape de filtration dudit échantillon sur un filtre à bactéries présentant des pores de 0,2 à 0,6 $\mu$m.

3. Utilisation conforme à la revendication 1, dans laquelle le temps pris par ladite détection desdites bactéries coliformes dans un échantillon est inférieur à 24 heures.

4. Utilisation conforme à la revendication 1, dans laquelle

a) ledit cocktail de détection comprend de l'eau peptonée, du milieu de MacConkey, du milieu DMEM, du sérum de veau foetal, du 4-nitro-phényl-$\beta$-D-glucuronide, du $\beta$-galactose et du D-glucose.

**Fig.1**

**Fig.2**

Fig.3

Fig.4B

Fig.4C

Fig. 5

30

Fig. 6

**Fig. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005202518 A
- US 2010062466 A
- US 2007281291 A
- EP 0254771 A

- WO 8904372 A
- WO 2016210436 A
- WO 2005012555 A